Europäisches Patentamt

(19) European Patent Office

Office européen des brevets

(11) EP 0 562 188 B1

(12) **EUROPEAN PATENT SPECIFICATION**

(45) Date of publication and mention
of the grant of the patent:
**30.09.1998 Bulletin 1998/40**

(51) Int. Cl.$^6$: **A01N 1/02**, A61K 9/00

(21) Application number: **92302726.2**

(22) Date of filing: **27.03.1992**

(54) **Composition for tissues to sustain viability and biological functions in surgery and storage**

Mittel für Gewebe zur Erhaltung der Lebensfähigkeit und biologische Funktionen in der Chirurgie und Lagerung

Composition pour les tissus destinée à soutenir la viabilité et les fonctions biologiques pendant la chirurgie et la stockage

(84) Designated Contracting States:
**CH DE FR GB IT LI**

(43) Date of publication of application:
**29.09.1993 Bulletin 1993/39**

(73) Proprietors:
• **Chen, Chung-Ho**
**Phoenix, Maryland 21131 (US)**
• **Chen, Sumi C.**
**Phoenix, Maryland 21131 (US)**

(72) Inventors:
• **Chen, Chung-Ho**
**Phoenix, Maryland 21131 (US)**

• **Chen, Sumi C.**
**Phoenix, Maryland 21131 (US)**

(74) Representative:
**Smart, Peter John**
**W.H. BECK, GREENER & CO**
**7 Stone Buildings**
**Lincoln's Inn**
**London WC2A 3SZ (GB)**

(56) References cited:
**EP-A- 0 215 138        WO-A-85/02975**
**WO-A-86/00227        FR-A- 2 468 366**
**US-A- 4 873 186        US-A- 5 073 492**

Printed by Xerox (UK) Business Services
2.16.3/3.4

## Description

The present invention relates to a number of compositions which are particularly useful as irrigating solutions in surgery, as cornea storage media, for preparing tissues for organ transplant and as organ culture media. The solutions provide a rich energy source for isolated tissue during storage and for tissues under surgery with concurrent suppression of lactic acid formation and accumulation in the cells. They may be used in ocular surgery and surgeries in general, although other uses, for example, topical application, and storage and rinsing of donor tissues prior to transplantation, are also contemplated.

Generally, an irrigating solution is used for the application on the external surface of the eyes and skin topically and on tissues in surgeries to rinse as well as to keep the operated tissues moist. However, in ocular surgeries, replacement of the aqueous and/or vitreous humors with the irrigating solution occurs as the consequence of ocular surgical procedures including corneal transplantation (penetrating keratoplasty), cataract extraction, intraocular lens implantation and vitrectomy. In these instances, the irrigating solution remains in the eyes after surgery until the components are either absorbed by the surrounding tissues or the solution is eventually equilibrated with body fluids, with subsequent clearance through the circulation. Thus, the irrigating solution used should not only be physiologically compatible, including tonicity and pH, but it should also contain components enabling the cells to sustain their viability and capability to perform physiological functions, at least until a full equilibration of the anterior chamber and the vitreous with the physiological body fluids is reached.

In ocular surgeries, components in the irrigating solution are of particular importance to the cornea and the lens. Both organs are avascular. The cornea obtains its nourishment mainly from the fluid in the anterior chamber, and to a lesser extent, from the tear and limbal vessel. The lens obtains its nourishment from fluids, both in the anterior chamber and in the vitreous. The retina, ciliary body and iris are vascularized tissues; they obtain their nourishment through the circulating plasma of the blood vessel network. Therefore, the components of the irrigating solution may not exert an effect on these tissues as significant as that on the cornea and the lens.

In the cornea, the monolayer endothelium lining the posterior surface contains fluid and ion transport sites which help maintain the cornea deturgescence and thereby the transparency. In the lens, the ion transport sites are located in the epithelium underneath the capsule. In general, the $Na^+$-$K^+$ ATPase is located in the cytoplasmic membrane which helps maintain intracellular $Na^+$ and $K^+$ contents at approximately 10 and 100 mM, respectively, and facilitates the transport against the extracellular salt concentration gradients of about 120 mM $Na^+$ and 10 mM $K^+$. Adequate energy is needed for the cells (and tissues) to perform the fluid and/or ion transport activities.

Glucose is the major energy source for mammalian cells. The cornea, lens and retina are very active glycolyzing tissues which utilize glucose and produce lactic acid, even under aerobic conditions. When the isolated cornea is stored in a medium containing glucose, excess lactic acid is formed and accumulated, resulting in an acidity that subsequently inhibits the metabolic activity of the tissue. In vivo, lactic acid formed in the vascularized tissue is cleared through the circulation, whereas that formed in the avascular cornea and lens is released into the anterior chamber and removed via the clearance mechanism of the circulation. In humans, lactic acid in the anterior chamber is maintained at a steady, low level of approximately 4.0 to 4.5 mM.

Glucose is an important and useful energy source for tissues when it is present in a solution for in vivo application. Utilization of glucose via glycolysis produces ATP at a high rate and is independent of $O_2$; however, it is not an effective energy-generating pathway, as only 2 moles of ATP are formed per mole of glucose utilized. Two moles of lactic acid are also formed. When the clearance mechanism of the operated ocular tissues is not sufficiently effective, lactic acid will be accumulated, resulting in a lower pH which, in turn, inhibits the metabolic activity of the tissues. In contrast, in tissues with a high mitochondrial population, a complete oxidation of glucose to $CO_2$ and $H_2O$ may occur, via mitochondria, generating as much as 38 moles of ATP per mole of glucose utilized, including the oxidation of two moles of NADH formed in glycolysis. Therefore, it is evident that utilization of substrates via oxidation in mitochondria is an effective energy-generating pathway when oxygen is available. Under this condition, there is no lactic acid accumulation. In addition, lactic acid accumulation may be diminished or minimized when anaerobic glycolysis is inhibited by an enhanced respiration via the Pasteur effect (Krebs, H.A., Essays Biochem., 8, 1, 1972).

Glucose cannot be used directly as a substrate in the mitochondria; it has to be converted first to pyruvate via glycolysis. Ketone bodies and precursors thereof (such as short chain fatty acids and ketogenic amino acids) are readily oxidized in the mitochondria, producing 32 molecules of ATP per acetyl moiety utilized. Therefore, ketone bodies and precursors thereof are energy-rich or energy-efficient molecules. Furthermore, oxidation of ketone bodies and precursors thereof results in an enhanced respiration which, in turn, inhibits anaerobic glycolysis via the Pasteur effect.

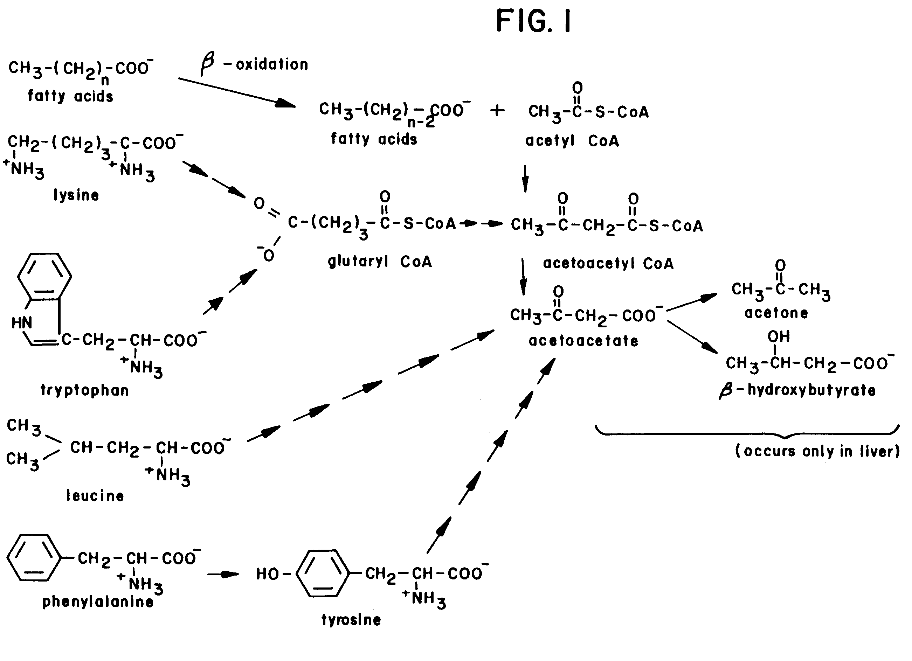

Ketone bodies are a collective term for acetone ($CH_3COCH_3$), acetoacetate ($CH_3COCH_2COO^-$) and β-hydroxybutyrate ($CH_3CHOHCH_2COO^-$). Pathways for the formation of ketone bodies from fatty acids and ketogenic amino acids are illustrated in FIGURE 1. Ketone bodies are metabolic products of fatty acids and ketogenic amino acids (including leucine, lysine, phenylalanine, tyrosine and tryptophan) in the tissues. In nature, fatty acids have a generalized formula of $CH_3(CH_2)_nCOOH$, where n is an even integer. Acetic acid is the smallest fatty acid molecule with n = 0. Ketone bodies are a stable form of the high energy metabolite of acetyl CoA for storage and delivery in the body. That is, ketone bodies are readily generated from fatty acids and ketogenic amino acids in vivo. Furthermore, β-hydroxybutyrate and acetoacetate are interconvertible through the catalysis of $NAD^+$-linked β-hydroxybutyrate dehydrogenase:

$$acetoacetate + NADH + H^+ = D-β-hydroxybutyrate + NAD^+$$

These indicate that ketone bodies, fatty acids, and ketogenic amino acids are interrelated molecules. When energy is needed, ketone bodies and precursors thereof are utilized in the tissues to form acetyl CoA, and are further oxidized to $CO_2$ and $H_2O$ in the mitochondria via the Krebs' cycle, yielding high energy in the form of ATP.

When ketone body precursors (fatty acids and ketogenic amino acids) are administered (to humans and animals), acetyl CoA is generated, which is further oxidized to yield a high level of ATP. If generation of acetyl CoA is in excess, ketone bodies are formed, with the major metabolic site being in the liver. Among those ketone bodies formed, acetone is not utilized and is excreted via breath, sweat and urine, whereas acetoacetate and β-hydroxybutyrate are delivered to the peripheral tissues where they are converted to acetyl CoA and are further oxidized in the mitochondria to $CO_2$ and $H_2O$ via acetyl CoA and citric acid cycle, yielding a rich energy for the peripheral tissues.

In the peripheral tissues, generation of acetoacetate from the ketogenic amino acids also takes place. There, acetoacetate is converted to β-hydroxybutyrate for storage if acetoacetate is in excess, or to acetyl CoA and further oxidized to $CO_2$ and $H_2O$ in the mitochondria to generate a high level of ATP. In the peripheral tissues, fatty acids are degraded to acetyl CoA via β-oxidation (see FIGURE 1), which is further oxidized to $CO_2$ and $H_2O$ in the mitochondria to generate high energy ATP.

The cornea, lens and retina are very active in glycolysis, which metabolize glucose to produce copious quantities of lactate. Glucose is an energy source for ocular tissues in vivo under normal conditions. The lactate formed is removed through the clearance mechanism of blood circulation. During or immediately following ocular surgery, the mechanism for the removal of lactate through circulation is not effective. When glucose is included in an irrigating solution as the energy source for ocular tissues in ocular surgery, it leads to excess accumulation of lactic acid in the anterior chamber and vitreous body. Use of glucose also leads to a lower extracellular pH, which in turn causes lactate accumulation in cells (and tissues) with subsequent inhibition of metabolic activity (Chen, C.H. and Chen, S.C., Arch. Biochem. Biophys., 276, 70, 1990). The problems can be overcome by addition of ketone bodies and precursors thereof in the irrigating solution as alternate fuel for the ocular tissues. The ketone bodies and precursors thereof are readily utilized in the ocular tissues to form acetyl CoA, with subsequent oxidation in the mitochondria to $CO_2$ and $H_2O$ via the Krebs' cycle, generating a high level of ATP. This process will inhibit glycolysis, via the Pasteur effect, and thereby suppress lactate production.

Moreover, ketone bodies are known to be the preferred fuel for the brain, muscle, and kidney during starvation (Olson, R.E., Nature, 195, 597, 1962; Bassenge, E., et al., Am J. Physiol., 208, 162 1965; Owen, O.E., et al J. Clin. Invest., 46, 1589, 1967; and Howkins, R.A. et al., Biochem. J., 122, 13 1971 and 125, 541, 1971).

The present invention now provides in a first aspect an isotonic irrigating solution of pH 7.3 to 7.5 having an osmolarity in the range of from 285 to 300 mOsM, comprising substantially bicarbonate free aqueous phosphate-buffered balanced salt solution, 5 to 10 mM glucose, and 5 to 30 mM β-hydroxybutyrate or a similarly effective amount of the ketone bodies and/or precursors thereof so as to form a composition that effectively meets requirements of ocular and other peripheral tissues under surgery for efficient physiological and biochemical functions, but lacking essential amino acids and vitamins that would reduce storage stability.

The ketone bodies are preferably β-hydroxybutyrate and acetoacetate ions. The β-hydroxybutyrate ions are preferably selected from the group consisting of D-isomer of β-hydroxybutyrate, a D- and L-racemic mixture of β-hydroxybutyrate and D- and L-isomer mixtures of β-hydroxybutyrate. The ketone body precursors may be short-chain fatty acids selected from the group consisting of acetic acid and butyric acid, or ketogenic amino acids selected from the group consisting of leucine, lysine, phenylalanine, tyrosine and tryptophan.

The concentration of the ketone body precursors may be in the range of 0.1-25 mM.

The solution may comprise one or more ketogenic amino acids each having a concentration in the range of 0.1-5.0 mM, and in total suitably having a concentration of 7.5-12.5 mM.

Short-chain fatty acids used as ketone body precursors may be acetic acid at a concentration in the range of 15-25 mM, or butyric acid at a concentration in the range of 5-15 mM.

Optionally, a portion of NaCl, in an amount of 15 to 35 mM, is replaced with an equivalent amount of Na salts of one or more sugar acids selected from the group consisting of D-glucuronic acid, D-galacturonic acid, D-mannuronic acid, D-gluconic acid and D-glucaric acid.

The viscosity of the solution may be increased by the addition of neutral polysaccharide dextrans with mass ranging from 40 to 500 kilodaltons in an amount of from 2 to 20% to form a composition that effectively meets requirements of ocular tissues for efficient physiological and biochemical functions with concurrent prevention of synechia in ocular surgery.

In a second aspect, the invention provides a cornea storage medium of pH 7.1 to 7.5, which is substantially free of bicarbonate and which comprises

phosphate buffered balanced salt solution,
5-10 mM glucose,
5-30 mM ketone bodies and or precursors thereof,
20 to 30 mM hepes buffer, and
preformulated minimum essential amino acids and vitamins, wherein the concentration of NaCl is limited to 70 to 100 mM and the osmolarity is adjusted to lie in the range of from 285 to 300 mOsM by the addition of mannitol, sucrose or dextran.

Preferably, the NaCl concentration is no more than 85 mM and preferably the osmolarity of the solution is adjusted to an isotonicity of 290 mOsM.

In such a cornea storage medium a portion of NaCl, in an amount of 15 to 35 mM, may be replaced with an equivalent amount of Na salts of one or more sugar acids selected from the group consisting of D-glucuronic acid, D-galacturonic acid, D-mannuronic acid, D-gluconic acid and D-glucaric acid. The storage medium may further comprise a synergistically effective mixture of VEGF, uridine, thymidine and serum-derived factor.

In a third aspect, the invention provides a formulation for the preparation of tissues for organ transplant comprising a solution according to the first aspect of the invention and a synergistically effective mixture of VEGF, uridine, thymidine and serum-derived factor. Such a formulation may be used for the preparation of tissues for cornea transplant.

In a further aspect, the invention provides an irrigation solution comprising a cornea storage medium according to the second aspect of the invention and a synergistically effective mixture of VEGF, uridine, thymidine and serum-derived factor.

In a fifth aspect, the invention provides an organ culture medium comprising a cornea storage medium according to the second aspect of the invention and a synergistically effective mixture of VEGF, uridine, thymidine and serum-derived factor.

The present compositions are not intended to function merely as an artificial fluid to replace ocular fluid in ocular surgeries, or body fluid in general surgeries, or as irrigating solutions per se. Rather, the purpose of the present compositions is to provide ketone bodies and precursors thereof as a high energy source to the isolated tissues during storage, and to tissues for a transitional period during surgery and the post-operation period, thereby enabling the cells to sustain their viability and capability to perform their physiological and biological functions, at least, for instance, in the ocular surgeries, until a full equilibration of the anterior chamber and the vitreous with the physiological body fluids is reached.

Thus, ketone bodies and precursors thereof and glucose in a balanced formulation are particularly useful for isolated tissues during storage and for peripheral tissues under surgery. Glucose is provided as the energy source for the tissues with very little or no mitochondria, such as the corneal stroma and the lens. Ketone bodies and precursors thereof are rich in energy; they are readily oxidized in the mitochondria of the peripheral tissues via the Krebs' cycle to form $CO_2$ and $H_2O$, with accumulation of no other metabolic wastes. Carbon dioxide formed is subsequently hydrated to form carbonic acid, which is further dissociated to form bicarbonate at pH 7.4. Bicarbonate has been suggested as necessary for the ion transport process across the corneal endothelium (Hodson, S., et al., J. Physiol., 263, 563, 1976). In addition, oxidation of ketone bodies and precursors thereof not only yields a high level of ATP, but also suppresses glycolysis via the Pasteur effect, resulting in inhibition of lactate production.

In the present invention, balanced salts are included in the ingredients to form a physiologically compatible solution for in vivo application. Phosphate in the ingredients is an element for oxidative phosphorylation in the ATP generation process when ketone bodies and precursors thereof are oxidized. In addition, phosphate also serves as a buffer, which has a high buffer capacity at the physiologic pH ranges, from pH 7.2 to pH 7.4.

The theory and intended applications of the present invention differ from the teaching of Veech as disclosed in U.S. Patent No. 4,663,289. Veech's invention is based on the theory that metabolic processes in living animal cells can be regulated by one or more of $[HCO_3^-]/[CO_2]$, $[\text{L-lactate}^-]/[\text{pyruvate}^-]$, and $[\beta\text{-hydroxybutyrate}]/[\text{acetoacetate}]$ couples with defined ratios, which is a purpose analogous to the buffer system of "weak acid/conjugated base" couple in regulating the pH. Veech's invention is based on several assumptions:

1. Both of the coupled substrates with defined ratios will be taken up by the cells or tissues.
2. The accumulated (in the tissues) substrates with defined ratios then remain in the living cells to regulate the metabolic process.
3. Substrate uptakes and metabolic regulation by these substrate couples are similar for all tissues.

Much of the experiments, on which Veech based his invention, were done with rat liver (Veech, et al., Biochem. J.,

115, 609, 1969; and Veech, et al., J. Biol. Chem., 254, 6538, 1979). The liver is the metabolic center in humans and animals. The metabolic process in the liver differs from that in the peripheral tissues. Therefore, the device for regulating the metabolic process may work in the liver, but it may not necessarily work in the peripheral tissues. For example, ketone bodies are formed in the liver through β-oxidation of fatty acids. The ketone bodies formed are not utilized in the liver, but are translocated to the peripheral tissues via the blood circulation. There, they are oxidized via the Krebs' cycle to $CO_2$ and $H_2O$, coupling to the formation of a high level of ATP (Principles of Biochemistry, Lehninger, Worth Publishers, Inc.). Thus, β-hydroxybutyrate is an efficient alternate fuel for the peripheral tissues, but not for the liver. The present invention utilizes the high ability of peripheral tissues such as the cornea and the lens to extract and metabolize β-hydroxybutyrate, without coupling to acetoacetate, to generate ATP with concurrent suppression of lactic acid formation and accumulation in the cells.

It is known that lactate formed is delivered into the blood by actively glycolyzing normal cells such as erythrocytes, skeletal muscle, cornea and retina, and is in turn extracted from the blood and metabolized by the liver and heart. Lactate is translocated across cytoplasmic membrane with pyruvate as the competitive inhibitor (Spencer and Lehninger, Biochem. J., 154, 405, 1976). Ketone bodies are formed and delivered into the blood by the liver and are in turn extracted from the blood and metabolized by the peripheral tissues such as corneas and lenses (Principles of Biochemistry, Lehninger, Worth Publishers, Inc.). The mechanism for transport of ketone bodies across the cytoplasmic membrane has not yet been established.

The metabolism of pyruvate, lactate, acetoacetate, and β-hydroxybutyrate varies with cells or tissues. The lactate dehydrogenase (LDH) isozyme in the heart and liver favors the oxidation of low concentration lactate to pyruvate; whereas LDH isozyme in the cornea, lens and retina favors the reduction of low concentration pyruvate to lactate. The β-hydroxybutyrate dehydrogenase (BDH) isozyme in liver favors the reduction of low concentration acetoacetate to β-hydroxybutyrate; whereas BDH isozyme in the peripheral tissues such as the cornea favors the oxidation of low concentration β-hydroxybutyrate to acetoacetate. Variations of the LDH and BDH isozymes in different tissues make it difficult to establish a generalized ratio of substrate-couple, such as [lactate]/[pyruvate] and [β-hydroxybutyrate]/[acetoacetate], for all tissues. The substrate uptake, continuous influxes of these chemicals from food-intake and other metabolic reactions, and removal of these chemicals through effluxes and other subsequent metabolic reactions make it more difficult to establish a generalized ratio of substrate-couple.

In the application of a medium for tissue storage, the teaching of Lindstrom et al. (U.S. Patent No. 4,695,536) is essentially the tissue culture technique that scientists in the tissue culture field have been using for years. Specifically, Lindstrom et al. teach a process of corneal storage comprising:

(a) mixing a corneal storage media including Gibco's minimum essential media of 50 ml essential media with Earls salts, 25 mM HEPES buffer without L-glutamine, 5 ml of L-glutamine (200 mM) at 1 percent final concentration of final media volume and antibiotics including 50-100 μg/ml of garamycin; serum from the group of calf serum, fetal calf serum or human serum; and an effective amount of high molecular weight molecule selected from a group consisting of chondroitin sulfate, sodium hyaluronate, keratan sulfate, sodium hyaluronate, keratan sulfate, polyvinyl-pyrolidone, methyl cellulose, hydroxy-prophylmethylcellulose, cellulose gum and dextran;
(b) filling a corneal storage container with the mixed media; and
(c) supporting a corneal-scleral rim in the container containing the media, whereby the media is kept at a temperature between 4°C to 34°C, thereby providing intermediate term storage and long term storage, and the system allowing shifting of temperature providing for transportation of tissue.

The teaching provides no mechanism to inhibit lactate production and accumulation when the isolated cornea is cultured or stored in the McCarey-Kaufman medium (tissue culture medium 199 plus 5% dextran; McCarey, B. and Kaufman, H., Invest. Ophthalmol., 13, 165, 1974), with or without supplement of calf serum (Lindstrom, et al., Am. J. Ophthalmol., 95, 869, 1977). In the teaching of McCarey, et al. (Invest. Ophthalmol., 13, 165, 1974), dextran is added to the tissue culture medium (TC 199) as a dehydrating agent, with which the active osmolarity of the medium becomes slightly hypertonic, about 305-340 mOsM. It is believed that dextran in this slightly hypertonic solution exerts a colloid osmotic pressure on the stored corneas, resulting in an artificial dehydrating effect. However, the presence of dextran in the medium has no inhibitory effect on lactate production and accumulation. In sharp contrast, the teaching of Chen et al. (U.S. Patent Ho. 4,873,186) provides a mechanism to inhibit lactate production and accumulation with concurrent generation of a high level of ATP in the tissue when the isolated cornea is incubated in Dulbecco's phosphate buffered salts solution (PBS) containing β-hydroxybutyrate, or stored in TC 199 (also known as Medium 199) containing β-hydroxybutyrate. Specifically, Chen et al. disclose a method of extending the time a surgical quality isolated cornea can be stored, which method comprises including in the corneal storage medium containing the stored cornea, an amount of at least one compound selected from the group consisting of (i) short chain fatty acids, (ii) ketone bodies and (iii) ketogenic amino acids or ketone body precursors capable of inhibiting lactate production by the isolated cornea, in an amount sufficient to inhibit the lactate production.

A corneal storage medium may be formulated by replacing the bicarbonate-balanced salts solution portion of the tissue culture medium, such as TC 199 with Hank's salts, with a composition according to the present invention. In this case, the NaCl concentration must be reduced to maintain a total combined concentration of anions and cations that is physiologically compatible. As a result, the active osmolarity becomes slightly hypotonic because components in the tissue culture medium, except NaCl, are not dissociated completely. The active osmolarity of the solution should be adjusted to an isotonicity of 290 mOsM, or in the range of 285-300 mOsM. Cytoplasmic membrane impermeable neutral saccharides, such as mannitol, sucrose, and dextran, may be used for the osmolarity adjustment. Their application results in no exertion of a colloid osmotic pressure on the stored corneas because of the isotonicity of the solution, and therefore, is distinctly different from the theory and intended application of dextran in the teaching of McCarey, et al., Invest. Ophthalmol., 13, 165, 1974).

β-Hydroxybutyrate is a reducing agent. In solution, it may be oxidized by $O_2$, especially at high temperature. Therefore, degassed water should be used to make the solution of the present composition. The resulting solution is stored under vacuum to protect β-hydroxybutyrate from oxidation by $O_2$, thereby extending the shelf-life. When the bottle is opened for clinical application, the solution will be re-equilibrated with the air and $O_2$ will be available for the biological functions of the tissues (or the cells).

Alternatively, an antioxidant of the group consisting of citrate, phenylalanine and vitamin E may be used to protect β-hydroxybutyrate from oxidation by $O_2$. The antioxidant is preferably citrate and the concentration of the citrate in the solution is preferably in the range of 8-12 mM. However, $O_2$ in the solution will also oxidize the antioxidant and render the antioxidant less effective in protecting β-hydroxybutyrate from oxidation by $O_2$ after extended storage. Thus, the process of using degassed $H_2O$ to make the solution of the present composition is a preferred method.

The invention will be further described and illustrated in respect of its preferred practice with reference to the accompanying drawings, in which:-

Figure 1 shows the pathways for the formation of ketone bodies from fatty acids and ketogenic amino acids.

Figure 2 is a reproduction of a photograph taken by a specular microscope of cat endothelium following anterior chamber irrigation with BSS as described in Figure 1.

Figure 3 is a reproduction of a photograph taken by a specular microscope of cat endothelium following anterior chamber irrigation with BSS-plus as described in Example 1.

Figure 4 is a reproduction of a photograph taken by a specular microscope of cat endothelium following anterior chamber irrigation with the present composition as described in Example 1.

Figure 5 is a "corneal thickness vs time" plot of in vitro deswelling of the isolated corneas in the presence of the present composition as described in Example 2.

Figure 6 is a "corneal thickness vs time" plot of in vitro deswelling of the isolated corneas in the presence of BSS-plus as described in Example 2.

Figure 7 is a "corneal thickness vs time" plot of in vitro deswelling of the grafted donor corneas with prior storage in the medium containing the present composition for 7 and 11 days at 4°C ad described in Example 3.

Figure 8 is a "corneal thickness vs time" plot of in vitro deswelling of a grafted donor cornea with prior storage in Optisol for 7 and 11 days at 4°C as described in Example 3.

Broadly described, the compositions of the present invention comprise ketone bodies and precursors thereof, glucose, and an aqueous phosphate-buffered balanced salt solution. The compositions utilise certain of the special features of their components, particularly the glucose and ketone bodies and/or precursors thereof as detailed above, so as to effectively meet the requirements of ocular and other peripheral tissues for efficient physiological and biochemical functions.

The present compositions containing ketone bodies and/or precursors thereof, glucose, and aqueous phosphate-buffered balanced salt solution thus satisfy the minium essential nutrient requirements of ocular tissues, both with or without high density of mitochondria (or no mitochondria) and of other tissues in general. Ocular and other peripheral tissues irrigated using the present composition, for example, following surgery, are capable of performing energy-dependent metabolic functions, such as fluid and ion transport and protein synthesis, and physiological functions such as maintaining a thin and clear cornea.

Such a composition thus provides, for example, a balanced and rich energy source for the peripheral tissues, typified by the three different tissue layers of the cornea. That is, it satisfies the need for the high respiratory activity of the endothelium, for the high glycolytic activity of the epithelium and the stroma, and for the attainment of an essential intracellular energy level for corneal fluid and ion transport.

Typical compositions which are suitable for use as an irrigating solution according to the first aspect of the present invention, including ranges of the components, are summarized in Table 1 below.

TABLE 1

| TYPICAL COMPOSITIONS OF THE PRESENT SOLUTION | | | |
|---|---|---|---|
| Components | Concentrations | | |
| | mg/ml | mM | Ranges |
| D,L-Sodium β-Hydroxybutyrate | 2.52 | 20 | 5 - 30 |
| Glucose | 0.99 | 5.5 | 5 - 10 |
| Sodium Chloride | 6.74 | 110 | 90 - 130 |
| Dibasic Potassium Phosphate (anhydrate) | 1.06 | 6.1 | 4.9 - 7.3 |
| Monobasic Sodium Phosphate (monohydrate) | 0.54 | 3.9 | 3.2 - 4.7 |
| Magnesium Chloride (hexahydrate) | 0.10 | 0.5 | 0.2 - 1.0 |
| Calcium Chloride (anhydrate) | 0.11 | 1 | 0.5 - 1.5 |
| pH is adjusted to 7.4 with HCl or NaOH. | | | |

Typical anions and cations contained in the present composition are shown in Table 2 below. Table 2 also shows the concentrations of these anions in the present composition. The calculated osmolarity of the the present composition is within the range of about 300 to about 320 mOsM if all the salts are fully dissociated. The actual osmolarity of the prepared solution of the present composition is in the range of about 285 to about 300 mOsM.

TABLE 2

| TYPICAL COMPOSITION OF IONS IN THE PRESENT SOLUTION | |
|---|---|
| Ions | Concentrations (mM) |
| $Na^+$ | 134 |
| $K^+$ | 12 |
| $Mg^{2+}$ | 0.5 |
| $Ca^{2+}$ | 1.0 |
| $Cl^-$ | 113 |
| Inorganic Phosphate (Pi) | 10 |
| β-Hydroxybutyrate | 20 |
| Glucose is a neutral molecule and is not included in Table 2. | |

According to one embodiment of the present invention, the ketone bodies of the composition are β-hydroxybutyrate and acetoacetate ions. The β-hydroxybutyrate ions are preferably selected from the group consisting of the D-isomer of β-hydroxybutyrate, a D- and L-racemic mixture of β-hydroxybutyrate and a D-and L-isomer mixture of β-hydroxybutyrate, and are most preferably the D-isomers of β-hydroxybutyrate. The concentration of the β-hydroxybutyrate in the solution is preferably in the range of 5-30 mM.

Preferred ketone body precursors of the present composition are short-chain fatty acids selected from the group consisting of acetic acid and butyric acid, and preferred ketogenic amino acids are selected from the group consisting of leucine, lysine, phenylalanine, tyrosine and tryptophan.

The concentration of the ketone body precursors is preferably in the range of 0.1-25 mM. The ketogenic amino acids preferably have a concentration in the range of 0.1-5.0 mM and most preferably have a concentration in the range of 1-2 mM. The ketogenic amino acids preferably have a combined total concentration of 7.5-12.5 mM, and most preferably, have a combined total concentration of 10 mM.

The preferred short-chain fatty acids are acetic acid, preferably at a concentration in the range of 15-25 mM, or butyric acid, preferably at a concentration in the range of 5-15 mM. The acetic acid is most preferably at a concentration of 20 mM and the butyric acid is most preferably at a concentration of 10 mM.

The pH of the present composition is preferably adjusted to 7.3-7.5.

De-gassed $H_2O$ is preferably used to make the present composition to protect β-hydroxybutyrate from oxidation by $O_2$, and thereby to extend the shelf-life of the solution at room temperature.

A portion of NaCl, preferably in an amount of 15 to 35 mM, or most preferably in an amount of 25 to 30 mM, may be replaced with an equivalent amount of $Na^+$ salts of one or more sugar acids including D-glucuronic acid, D-galacturonic acid, D-mannuronic acid, D-gluconic acid and D-glucaric acid.

The viscosity of the present composition is preferably increased by the addition of neutral polysaccharide dextrans with mass ranging from 40 to 500 kilodaltons in an amount sufficient to form a composition that effectively meets the requirements of ocular tissues for efficient physiological and biochemical functions with concurrent prevention of synechia in ocular surgery. The preferred concentration of neutral high molecular mass dextran is in the range of 2% - 20%.

In its second aspect the invention includes a cornea storage medium wherein the balanced salt solution of a tissue culture medium is replaced with the present composition; the NaCl concentration is reduced to 70-100 mM, or most preferably to 85 mM, to maintain the same combined total of cation and anion concentration in the solution; and the active osmolarity of the solution is adjusted to an isotonicity of 285-300 mOsM or, most preferably, to 290 mOsM; and mannitol, sucrose or dextran are added if and as needed. The tissue culture medium is preferably TC 199 with Hank's salts or Eagle's minimum essential medium with Hank's salts.

The pH of the cornea storage medium is within the range of 7.10-7.50, and most preferably within the range of 7.25-7.40.

A portion of NaCl, in an amount of 15 to 35 mM, or preferably in an amount of 25 to 30 mM, may be replaced with an equivalent amount of Na salts of one or more sugar acids including D-glucuronic acid, D-galacturonic acid, D-mannuronic acid, D-gluconic acid and D-glucaric acid.

The cornea storage medium may be one wherein the phosphate-buffered salt solution of the present composition replaces a bicarbonate-buffered balanced salt solution of a cornea storage composition comprising a tissue culture medium suitable for storing corneas but in which undesired lactate production would normally occur. The cornea storage medium may additionally include at least one compound selected from the group consisting of short-chain fatty acids and ketone bodies capable of inhibiting lactate production by the corneas, the compound being present in an amount sufficient to inhibit lactate production.

The invention includes a cornea storage composition comprising a cornea storage medium of the second aspect of the invention and a synergistically effective mixture of VEGF, uridine, thymidine and serum-derived factor.

A method of preparing a solution according to the first aspect of the present invention comprises mixing together glucose and ketone bodies and/or precursors thereof in a degassed aqueous phosphate-buffered salt solution in amounts sufficient to form a composition that effectively meets the requirements of ocular and other peripheral tissues for efficient physiological and biochemical functions, as detailed above. The solution is then bottled under vacuum to insure a complete elimination of $O_2$ from the solution to protect β-hydroxybutyrate from oxidation during extended storage at room temperature. If the solution is to be used immediately or within a short time, the vacuum-bottling procedure is not necessary, although helpful.

Because $Ca^{2+}$ and $Mg^{2+}$ at high concentrations form phosphate salt precipitate, it is preferable that all ingredients except $CaCl_2$ and $MgCl_2$ are dissolved in adequate degassed $H_2O$ first, preferably 90% - 95% of total volume. The solution is thoroughly mixed and the pH is adjusted to within the range of 7.3 to 7.5 with 1 N glucuronic acid or NaOH. Then, $CaCl_2$ and $MgCl_2$, in 0.5 M stock solutions, or in ranges from 0.2 to 1.0 M, are added. The pH is checked and adjusted if necessary, to 7.3 to 7.5, preferably pH 7.4. Finally, $H_2O$ is added to make up the volume. Preferably, deionized double-distilled $H_2O$ is used for the solution preparation. All the components used should be reagent grade.

A number of variations are possible when preparing the present irrigation solution. Some examples of possible variations are given below.

1. The phosphate buffer can be prepared using dibasic potassium phosphate and monobasic potassium phosphate. In this case, total potassium ions are increased to 15 mM.

2. The phosphate buffer can be prepared using dibasic sodium phosphate and monobasic sodium phosphate. In this case, 8 to 12 mM KCl should be added, and NaCl is subtracted by 5 mM.

3. The phosphate buffer can be prepared using any other phosphate salts and phosphoric acid, with an appropriate titration to pH 7.4. In any case, $K^+$ should have a final concentration of 8 to 12 mM. The pH can vary from 7.3 to 7.5.

4. The chloride concentration should be maintained within the physiological range of 90 - 105 mM by replacing a portion of NaCl with $Na^+$ salts of one or more of sugar acids including D-glucuronic acid, D-galacturonic acid, D-mannuronic acid, D-gluconic acid and D-glucaric acid.

5. Sodium salts of one or more ketogenic amino acids including leucine, lycine, phenylalanine, tryptophan, and tyrosine, at concentrations from 0.1 to 5.0 mM, preferably 1 to 2 mM, can be added as components of the irrigating solution. An equivalent amount of NaCl is subtracted to adjust $Na^+$ concentration.

6. A portion of β-hydroxybutyrate can be replaced with two equivalent amounts of acetate, the smallest molecule of the ketone body precursor short chain fatty acids.

7. Sodium salts of other short chain fatty acids, such as butyric acid and caproic acid, at 10 mM, or in ranges from 5 to 15 mM, can also be used. However, their low solubility in the aqueous solution may limit their application.

8. Although β-hydroxybutyrate can be replaced by either acetate or acetoacetate, β-hydroxybutyrate is preferred. β-Hydroxybutyrate is stable and cost-efficient. It is also readily utilized by the cells, and it is more energy-efficient than acetoacetate. One mole of β-hydroxybutyrate will generate 64 moles of ATP and one mole of NADH, which can further generate 3 moles of ATP.

9. The D-isomer of β-hydroxybutyrate is a preferred component of the present composition because only D-isomer is utilized in the cells. However, the D-, L- racemate or D- and L-isomer mixtures are cost-efficient and readily available commercially, and can be used.

10. If necessary, saccharides such as mannitol and sucrose or neutral polysaccharides such as dextran can be used to adjust the active osmolarity of the solution to 290 mOsM or in the range of about 285 to 300 mOsM. Such an adjustment results in no changes in the $Na^+$ and $Cl^-$ concentrations. If both the $Na^+$ and the $Cl^-$ concentrations are too high, NaCl should be replaced with an equal equivalent amount of saccharides.

11. Neutral high molecular mass dextran, preferably 70-500 kilodaltons, at 5% or in the ranges from 2% to 20%, can be used to increase the viscosity of the solution. The highly viscous composition is useful for application in the ocular surgeries to prevent synechia due to surgical wounds, in which the lens or the iris is adhered to the corneal endothelium, or the iris is adhered to the lens. In this case, NaCl is subtracted by 2.5 mM per 1% of dextran added.

The composition of the first aspect of the invention is formulated with the minimum of essential components for use primarily to enable ocular and other peripheral tissues (or cells) to sustain their viability and capability to perform their physiological functions during the transition period following intraocular surgeries including, for example, corneal transplantation, intraocular lens implantation, cataract extraction and vitrectomy. The transition period is defined as the time between surgery and a full equilibration of the solution in the anterior chamber and/or the vitreous with the physiological fluids from the body. As noted above, the present composition may also be used, for example, for skin and ophthalmic topical application, for storage and rinsing of donor tissues prior to transplantation, and for surgeries in general.

The present composition contains a physiologically compatible salt solution, with concentration ranges similar to those found in blood plasma. According to the present invention, phosphate (typically 10 mM) is used as both a buffer and a substrate for phosphorylation of ADP to form ATP in energy metabolism. Phosphate has a strong buffer capacity at pH 7.2 -7.4. Bicarbonate is used in other irrigation solutions currently available in the market (such as BSS-plus, manufactured and marketed by Alcon, Fort Worth, Texas). However, bicarbonate is not utilized as a component in the present composition because, when a bicarbonate buffer is used, the pH of the solution varies with $CO_2$ partial pressure ($P_{CO2}$). Furthermore, although exogenous bicarbonate is not added, bicarbonate is formed via the respiration (or oxidative phosphorylation) of the tissues in situ. In this process, the substrates are oxidized (in the mitochondria) to form $CO_2$ and $H_2O$. $CO_2$ formed is then hydrated to form $H_2CO_3$, which is rapidly dissociated to form $HCO_3^-$. In the rabbit cornea, $CO_2$ is produced at a rate of about 1.3 μmoles/cm$^2$/hr. In human corneas, the rate is about 30 to 50% higher.

D-β-Hydroxybutyrate is readily oxidized in the mitochondria to form acetoacetate and NADH via the catalysis of β-hydroxybutyrate dehydrogenase. Acetoacetate further reacts with succinyl CoA to form acetoacetyl CoA, which is then further degraded, with the catalysis of thiophorase, to form acetyl CoA. Fatty acids are utilized in the cells via β-oxidation, to form acetyl CoA. Ketogenic amino acids are also catabolized in the cells to yield acetoacetate or acetoacetyl CoA and then acetyl CoA. Acetyl CoA from these reactions are then further oxidized to $CO_2$ and $H_2O$ via the citric acid cycle in the mitochondria. Pathways of ketone body formation from fatty acid and ketogenic amino acids are briefly illustrated in FIGURE 1. The oxidation of acetyl CoA is an energy-efficient process, 30 moles of ATP and 2 moles of GTP (or a total of 32 moles of ATP) being formed per mole of acetyl CoA utilized. Inorganic phosphate in the irrigating solution provides for oxidative phosphorylation, which is coupled to electron transport, to react with ADP to form ATP.

For glucose consumed in the cornea, about 47% is via glycolysis and 53% via respiration (Chen and Chen, Arch. Biochem. Biophys., 276, 70, 1990), as may be seen from Table 3 below.

TABLE 3

| METABOLIC ACTIVITY IN CORNEAL TISSUE LAYERS | | |
|---|---|---|
| Tissue Layers | Glucose Consumption Rate (μmol/cm$^2$/hr) | $\dfrac{\text{Lactate Formation}}{\text{Pyruvate Oxidation}}$ Ratio |
| Epithelium | 0.185 ± 0.02 (48%) | 1:1 |
| Stroma | 0.096 ± 0.01 (25%) | 2:1 |

TABLE 3 (continued)

| METABOLIC ACTIVITY IN CORNEAL TISSUE LAYERS | | |
|---|---|---|
| Tissue Layers | Glucose Consumption Rate ($\mu$mol/cm$^2$/hr) | $\dfrac{\text{Lactate Formation}}{\text{Pyruvate Oxidation}}$ Ratio |
| Endothelium | $0.107 \pm 0.01$ (28%) | 1:2 |

The percent glucose consumption in the corneal tissue layers is shown in parenthesis in Table 3 above. The stroma consumes glucose via glycolysis at a rate about twice that of respiration. In the lens, mitochondria are located only in the epithelium. Thus, glucose is an important energy source in the stroma and the lens, especially cells in the lens nucleus. In these cells, there are very little or no mitochondria to utilize acetyl CoA. In other ocular tissues, such as the corneal endothelium and photoreceptors, where mitochondrial population is high, ketone bodies and precursors thereof are energy-efficient substrates. In addition, in these tissues, acetyl CoA oxidation elicits an enhanced respiration which, in turn, inhibits anaerobic glycolysis via the Pasteur effect.

Thus, the present composition containing ketone bodies and/or precursors thereof, glucose, and a phosphate-buffered balanced salt solution satisfies the minimum essential nutrient requirements of ocular tissues, both with or without high density of mitochondria, or no mitochondrium, and of other peripheral tissues in general.

The composition of the present invention can be used as a simplified version, suitable for short term application, of the cornea storage composition disclosed by Chen et al. in U.S. Patent No. 4,873,186 (discussed above). According to the teaching of Chen et al., at least one compound selected from the group consisting of short chain fatty acids and ketone bodies is mixed with tissue culture medium and used for the storage of the isolated cornea to generate high energy with concurrent inhibition of lactate production by the corneas.

According to the second aspect of the present invention, a cornea storage medium may be prepared which comprises the composition of the first aspect of the present invention, essential amino acids and essential vitamins of either Eagle's (Science, 130:432, 1959; Proc. Soc. Exp. Biol. Med., 89:362, 1955), Dulbecco's (Virology, 8:396,1959 and 12:185, 1960) or Daniel's (Proc. Soc. Exp. Biol. Med., 127:919, 1968). It is helpful to add Hepes (N-2-hydroxyethylpiperazine-N-2-ethanesulfonic acid) buffer at 20 to 30 mM to strengthen the buffer capacity. Preformulated minium essential amino acids and essential vitamins are commercially available. Because essential amino acids and vitamins in solutions in general have a relatively short shelf life and the solution has to be kept cold and be used within a short period of time, normally within 6-12 months, it is not necessary, although it is helpful, to use degassed $H_2O$ to make the present composition as used in the corneal storage medium.

A cornea storage medium may be formulated by replacing the balanced salt solution of a tissue culture medium, such as TC 199 and Eagle's minimum essential medium with Hank's salts, with a composition according to the first aspect of the present invention. NaCl concentration is reduced from 115 mM to within the range of 70 to 100 mM, so that the combined total of cations and anions in the medium are maintained at a physiological compatible level. The solution should be maintained at an isotonicity of 290 mOsM, or in the range of about 285-300 mOsM. The active osmolarity may be adjusted with saccharides such as mannitol and sucrose or neutral, high molecular mass polysaccharides such as 40 and 70 kilodalton dextrans.

A cornea storage medium may also be prepared by replacing the bicarbonate-buffered balanced salt solution of the cornea storage composition of the teaching of Chen et al. in U.S. Patent No. 4,873,186 (discussed above) with the phosphate-buffered balanced salt solution of the composition of the present invention. It is preferable to maintain the Cl concentration within the physiological range of 90 to 105 mM by replacing a portion of the NaCl in an amount of 15 to 35 mM, preferably 25 to 30 mM, with an equivalent amount of sodium salts of one or more of sugar acids including D-glucuronic acid, D-galacturonic acid, D-mannuronic acid, D-gluconic acid and D-glucaric acid. According to the teaching of Chen et al., at least one compound selected from the group consisting of short chain fatty acids and ketone bodies is mixed with tissue culture medium and used for corneal storage. Bicarbonate-buffered balanced salt solution is a portion of the tissue culture medium ingredients.

Cornea storage compositions described above may be combined with the synergistic composition described by Chen et al. (U.S. Patent No. 5,073,492). According to the teaching of Chen et al., the synergistic composition consists essentially of a synergistically effective mixture of vascular endothelial growth factor (VEGF), uridine, thymidine and serum-derived factor (SDF). The stoichiometric ratio of the uridine to thymidine is 10 $\mu$M uridine to 0.5 $\mu$M thymidine. Typically, dialysed fetal bovine retinal extract (0.1 mg/ml) and dialysed fetal bovine serum (3 mg/ml) are used as the sources of VEGF and SDF, respectively.

A composition of the first aspect of the present invention may also be combined with the synergistic composition of Chen et al. (U.S. Patent No. 5,073,492), as described above, to compose a formulation for the preparation of tissues for organ transplantation, including corneal transplantation.

A cornea storage medium as described above may be combined with synergistic composition of Chen et al. (U.S.

Patent No. 5,073,492), to form an irrigation solution for topical application to stimulate endothelial cell regeneration or to form an organ culture medium for application to facilitate the corneal endothelium replacement process prior to corneal transplantation.

A solution according to the first aspect of the present invention may be prepared by dissolving all ingredients in exact weights as indicated in the formula, except $Ca^{2+}$ and $Mg^{2+}$ salts, in degassed double distilled water with a volume equal to about 95% of total. The pH of the solution is adjusted to 7.4 with either glucuronic acid or NaOH, if necessary. Then $Ca^{2+}$ and $Mg^{2+}$ salts are added, followed with degassed double distilled water to end up the volume. The pH is rechecked and adjusted if necessary. The solution is then sterilised by appropriate means, such as through the use of an autoclave or filtration through a 0.22 µm filter (Nalge Co., Rochester, N.Y.). Then the solution is bottled under vacuum to insure a complete elimination of $O_2$ from the solution to protect β-hydroxybutyrate from oxidation by $O_2$.

When the composition of the present invention is used as part of a more complex formulation, such as has been described above, it is preferable to first prepare a concentrated stock solution with $Ca^{2+}$ and $Mg^{2+}$. Then, an exact, calculated volume is measured and combined with another part of the components, and double distilled water is added to make up about 95% of volume. $Ca^{2+}$ and $Mg^{2+}$ are added and the pH is rechecked and readjusted, if necessary. The stock solution is preferably, two to five times concentrate. If not used immediately, the stock solution should be sterilised by filtering through 0.22 or 0.45 µm membrane filters such as Nalgene (Nalge Co., Rochester N.Y.), preferably 0.22 µm membrane filters. The sterilised solution should be kept in a refrigerator at 4°C. Because the solution contains essential amino acids, vitamins and other components that have a relatively short shelf life, the solution is kept at 4°C and must be used within a short period of time, normally in about 6-12 months. Therefore, it is not necessary, although it is helpful, to use degassed $H_2O$ to prepare the composition of the present invention for inclusion in the formulation of these solutions.

If the composition of the present invention is combined with the synergistic composition, $Mg^{2+}$ should be omitted because the synergistic composition contains an adequate quantity of $Mg^{2+}$.

The present invention will be illustrated in detail in the following examples. The examples are included for illustrative purposes and should not be considered to limit the present invention.

EXAMPLE 1

The efficacy of a composition of the invention as a balanced energy source was compared to BSS and BSS-plus (manufactured by Alcon Laboratories, Inc., Fort Worth, Texas), two of the most widely used ophthalmic irrigating solutions at the present time. (Reportedly, BSS-plus is more effective than BSS.) Oxidised glutathione, a tripeptide, is present in BSS-plus. Glutathione has been suggested as having a beneficial effect on the fluid transport of the cornea according to Dikstein, et al. (J. Physiol. 221, 29, 1972).

The composition of the present invention as shown in Table 1, with the exception of the replacement of 10 mM β-hydroxybutyrate with 20 mM acetate and the addition of 10 mM citrate as antioxidant, was used for the experiments described in this example. Variations in concentrations of these components within the ranges as indicated in Table 1 are similarly effective. However, the active osmolarity of the solution should be maintained in the range of from about 285 to 300 mOsM, preferably at 290 mOsM. Because the purity of the salts may vary from lot to lot, the active osmolarity of the solution may vary from preparation to preparation. The osmolarity may be adjusted with the addition of mannitol if it is too low, or by replacing NaCl with sodium glucuronate or other $Na^+$ salts of sugar of sugar acids if it is too high.

According to the procedure of the experiment, two incisions, each about 2 mm in length, were made in the corneas of 6 cat eyes. A 25-gauge cannula was then placed in the anterior chamber through one of the incisions in each cornea. The solution of the present invention exemplified by Table 1 above, with the only exception being the replacement of 10 mM β-hydroxybutyrate with 20 mM acetate and the addition of 10 mM citrate as antioxidant, was then infused in the anterior chamber at a rate of about 7 ml/min, at a pressure of about 15 mm Hg, for a total of 1 liter per cornea.

For comparative purposes, the identical procedure was performed with the only exception being the replacement of the present solution with, in one case, BSS, and in another case, BSS-plus.

All corneas were examined at 1, 2 and 6 days following infusion. Endothelial morphism was examined using a specular microscope, and corneal thickness was measured using a digital pachymeter.

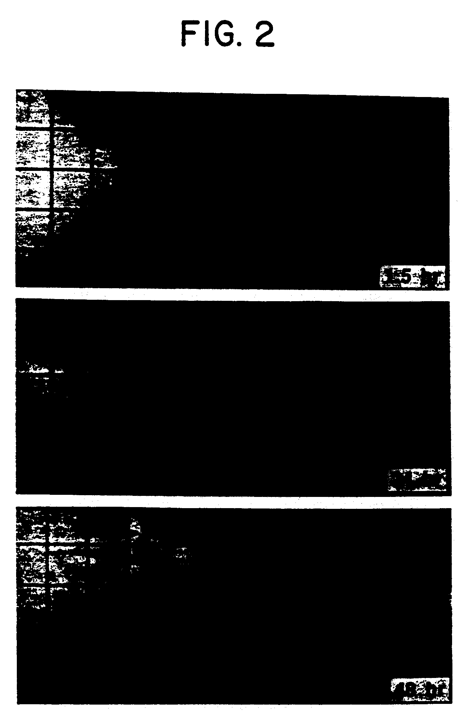

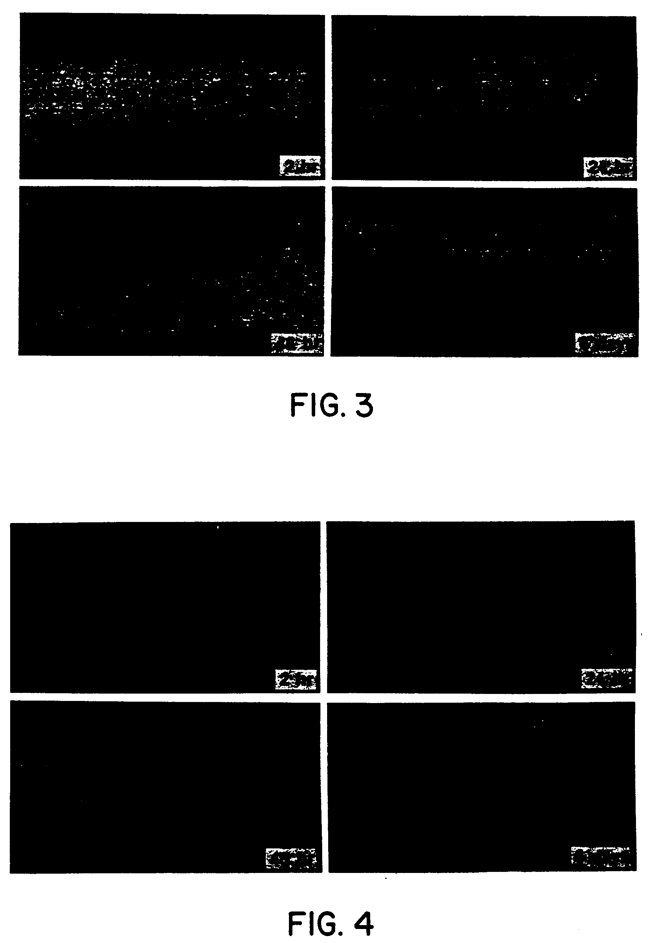

As shown in FIGURE 2, the corneas infused with BSS exhibited extensive endothelial cell losses, endothelial pleomorphism, and corneal swelling on day 1 following infusion. As shown in FIGURES 3 and 4, respectively, on day 1 following infusion with BSS-plus or the present irrigation solution, the shape of endothelial cells remained polygonal (a normal shape), and the corneas were of normal thickness. When examined on day 6 following infusion, the corneas infused with the present solution (FIGURE 4) were comparable or slightly better than those infused with BSS-plus (FIGURE 3), in terms of endothelial pleomorphism and corneal thickness.

The above results show that, in terms of maintaining an intact endothelium and a thin and clear cornea, the present irrigation solution is better than BSS-plus.

It is further noted that in this animal experiment, the continuous infusion of the testing solution into the anterior chamber was performed over a period of time which was much longer than that which takes place in ocular surgery under normal conditions. It is therefore concluded that the present composition has an efficacious effect on ocular tissues in clinical applications.

## EXAMPLE 2

It is known that the endothelium containing ion and fluid pump sites help maintain the cornea at a highly dehydrated state, and thereby the transparency. The fluid and ion transport of the endothelium is an energy-dependent process. When the donor dies (or the cornea is isolated) and is kept at 4°C for a period of time, the cornea will swell because of inadequate energy out-put at 4°C for the cornea to deturgesce. The cornea will deswell when the energy out-put is increased by raising the temperature of the bathing solution. The process is called temperature reversal. The deturgescence activity of the isolated cornea on the basis of temperature reversal is, therefore, a measurement of the barrier, fluid pump, and metabolic functions of the cornea as well as the efficacy of exogenous metabolites for the cornea to perform these functions.

According to the procedures of the experiments, New Zealand albino male rabbits, weighing 2.5 to 3.0 kg, were killed by an overdose intracardiac injection of pentobarbital, and kept in a cold room at 4°C with eyes taped shut for, in one case, 24 hours, and, in another case, 48 hours to induce corneal swelling and to elicit a reduced metabolic activity. The corneas with 1.5 mm scleral rim were then excised, briefly rinsed in Dulbecco's PBS, and placed in a Cooper Vision viewing chamber containing 22 ml of the solution of the present invention exemplified by Table 1 above. Corneal thickness was measured using a digital pachymeter attached to a specular microscope. "Corneal thickness vs time" was plotted, and corneal deturgescence activity in terms of tissue deswelling rate was determined.

For comparative purposes, the identical procedure was performed with the only exception being the replacement of the present solution with BSS-plus.

It is further noted that the experimental procedure resembles the in vivo deturgescent process of donor corneas after being grafted to the recipients.

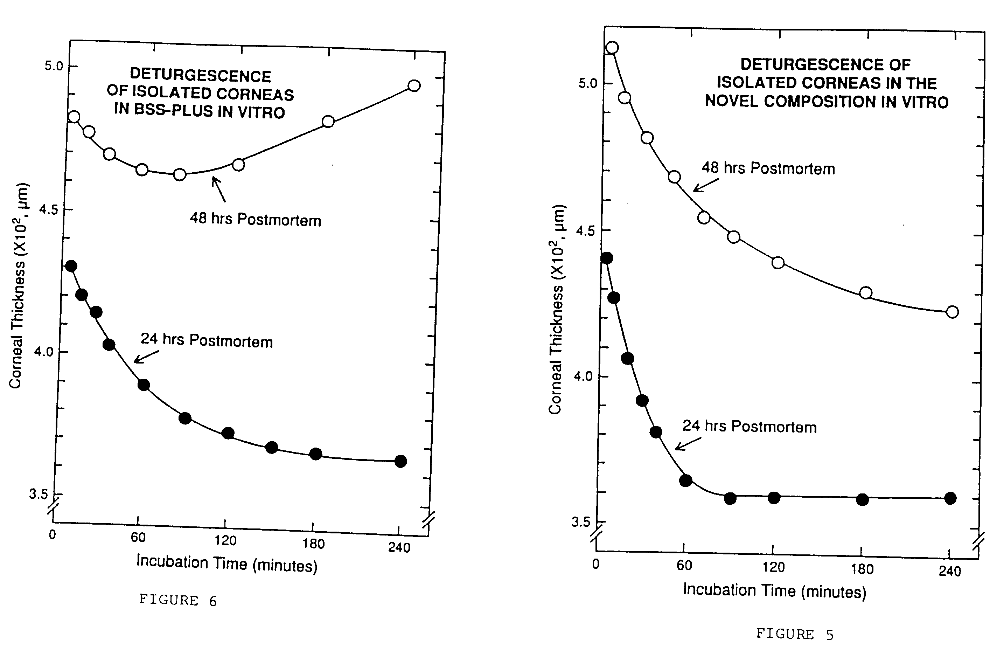

FIGURE 5 shows the typical "cornea thickness vs time" plots of rabbit corneas that deswelled in vitro at room temperature (22°C-23°C) in the solution of the present invention exemplified by Table 1 above. The tissues were isolated from animals at 24 or 48 hours after death. Deturgescence activity in terms of deswelling rate for the corneas with 24 hours and 48 hours postmortem was measured (six corneas each) to be $137.3 \pm 5.4$ μm/hr and $63.3 \pm 6.7$ μm/hr, respectively.

As shown in FIGURE 6, the isolated corneas in the presence of BSS-plus deswelled at rates of $89.7 \pm 6.4$ μm/hr and $30.3 \pm 3.8$ μm/hr for tissues isolated 24 hours and 48 hours after animal death (six corneas each), respectively. The rates are respectively 35% and 50% lower than those observed in the presence of the present solution (FIGURE 5).

The above results show that, in terms of efficacy as an energy source for tissues (and cells) to perform physiological and biological functions, the present composition is better than BSS plus. The results lend further support to the findings of the experiments described in Example 1.

## EXAMPLE 3

The teaching of Chen et al. (U.S. Patent No. 4,873,186, discussed above) revealed that, when β-hydroxybutyrate was mixed with tissue culture medium and was used to store the isolated cornea, a peripheral tissue, a high level of energy in terms of ATP was generated with concurrent inhibition of lactate production and accumulation in the stored tissue. The efficacy of a corneal storage medium containing the composition of the present invention for preserving viability and the endothelial pump function of the donor cornea in vivo was compared to Optisol (manufactured by Chiron, Irvine, CA). (Optisol is reported to be one of the best corneal storage media presently available in the market.) Chondroitin sulfate and dextran are present in the Optisol as dehydrating agents in amounts sufficient to exert a colloid osmotic pressure on the tissues.

The corneal storage medium for the experiments was prepared by replacing the bicarbonate and balanced salt solution of TC 199 with the composition of the present invention, with typical compositions and concentrations exemplified in Table 1, with the only exceptions being the omission of glucose and reduction of NaCl by 15 mM. TC 199 already contains glucose; therefore no additional glucose is needed. The reduction of NaCl by 15 mM is to maintain the same combined total of cation and anion concentrations after the bicarbonate and balanced salt solution of TC 199 are replaced with the composition of the present invention. Variations in concentrations of these components within the ranges as indicated in Table 1 are similarly effective. However, the active osmolarity of the solution should be maintained at an isotonicity of 290 mOsM, or in the ranges of about 285 to 300 mOsM, so that the solution will exert no osmotic effect on the tissue. The active osmolarity may be adjusted with saccharides such as mannitol and sucrose or neutral polysaccharide dextran.

The solution was sterilized by filtering through 0.22 μm Nalgene (Nalge Co., Rochester, N.Y.) filter membrane, placed (20 ml) in a 25 ml scintillation vial, and kept refrigerated at 4°C.

According to the procedures of the experiments, New Zealand albino male rabbits, weighing 3.0 to 3.5 kg, were killed by an overdose intracardiac injection of pentobarbital, and the corneas with 1.5 mm scleral rims were isolated. For comparative purposes, one of the pair of corneas from the same donor was stored in the corneal storage medium containing the composition of the present invention and another one was stored in Optisol. After storage for, in one case, 7 days and, in another case, 11 days, the donor cornea was removed from the storage medium, and briefly rinsed with Dulbecco's PBS. A 7.5 mm donor corneal button was cut with a trephine and transplanted to a recipient rabbit. The transplantation procedure took about 30-45 minutes. Donor corneal thickness was measured immediately after grafting, and then every 30-60 minutes for up to 7 hours, once to twice daily for one week and once a week for 4 weeks.

In general, isolated corneas swell during storage at 4°C, but this is reversible if the viability, cellular metabolic activity, and endothelium fluid pump function of the stored corneas are preserved. When grafted to a recipient, a donor cornea with well-preserved biological-functions will deturgesce rapidly, and clarity of the graft is readily attainable. If not well-preserved, the donor cornea will deturgesce at a lower rate, or even swell after grafting.

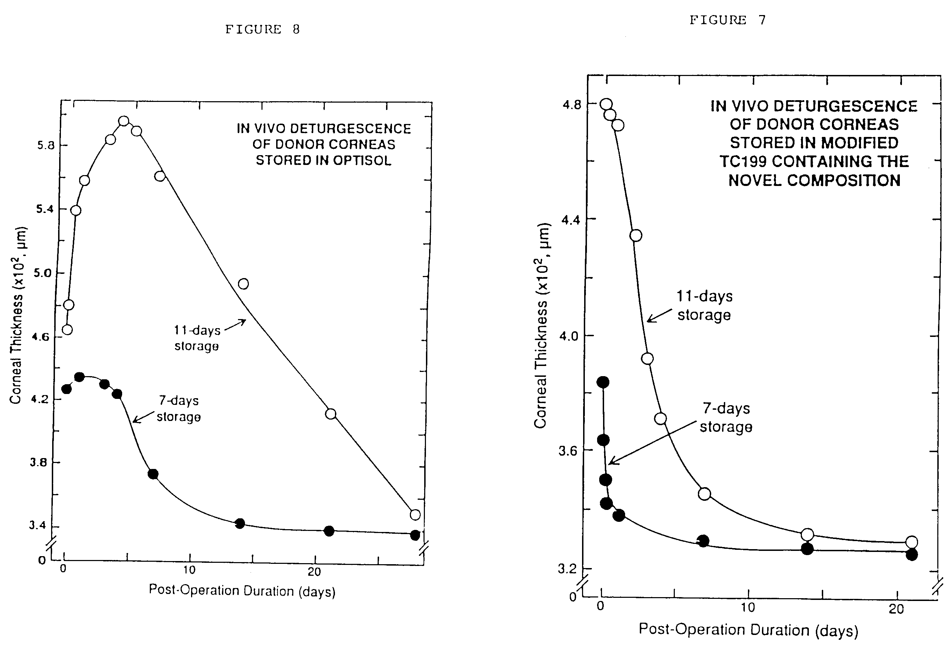

As illustrated in FIGURE 7, after transplantation the donor cornea that is stored in the medium containing the composition of the present invention for 7 days deturgesces rapidly, at about 16.2 $\mu$m/hr (average of 4 corneal grafts), and the cornea stored for 11 days deturgesces at a slower rate, at about 1.4 $\mu$m/hr (average of 4 corneal grafts). After transplantation, half-time ($t_{1/2}$) for the donor corneas stored for 7 and 11 days to return to the normal thickness of about 340-370 $\mu$m is established (4 grafts each) to be about 2.4 and 33.6 hours, respectively (Table 4).

In sharp contrast, as shown in FIGURE 8, the donor corneas stored in Optisol for 7 days exhibit a moderate swelling for about 3 hours after transplantation, and then deswell at an estimated rate of about 0.5 $\mu$m/hr (average of 4 corneal grafts). The donor corneas stored in Optisol for 11 days show extensive swelling after grafting for about 5 hours, and then deswell at an estimated rate of about 0.4 $\mu$m/hr (average of 4 corneal grafts). After transplantation, $t_{1/2}$ for the donor corneas stored in Optisol for 7 and 11 days to return to a normal thickness of about 340-370 $\mu$m is estimated (4 grafts each) to be about 133 and 311 hours, respectively (Table 4).

TABLE 4

| COMPARISON OF THE EFFICACY OF THE MEDIUM CONTAINING THE PRESENT COMPOSITION WITH THAT OF OPTISOL FOR DONOR CORNEAL STORAGE | | | |
|---|---|---|---|
| Parameters | Storage Duration (days) | Storage Media | |
| | | Present Invention | Optisol |
| In Vivo Deturgescent Rate ($\mu$m/hr) | 7 | 16.2 ± 8.5 | 0.51 ± 0.06 |
| | 11 | 1.4 ± 0.2 | 0.42 ± 0.07 |
| Half-time for Donor Cornea to Return to Normal Thickness After Transplantation (hours) | 7 | 2.4 ± 1.8 | 133.2 ± 14.3 |
| | 11 | 33.6 ± 14.4 | 311.2 ± 60.0 |
| Four corneas are used in each experiment. | | | |

The results obtained from these in vivo experiments explicitly show that, in terms of preservation of corneal tissue viability and corneal endothelial fluid pump function, the corneal storage medium containing the composition of the present invention is significantly better than Optisol.

Thus, the experiments described in Examples 1, 2, and 3 demonstrate that the present invention is a unique composition. It is clearly evident that the composition is particularly useful, for both in vitro and in vivo applications, as an energy source for peripheral tissues to perform their physiological and biological functions, and to preserve tissue viability.

In addition to its clinical properties, the present composition has the following qualities not possessed by other irrigating solutions such as BSS-plus. For example:

1) The present composition has a mechanism to prevent lactate formation and accumulation in the tissues while providing a rich energy source for the cells.

2) The present composition is chemically more stable than BSS-plus, and therefore has a longer shelf-life.

3) The present composition solution costs less to manufacture than does BSS-plus.

4) The buffer (phosphate buffer) of the present composition is stable and has a high capacity. BSS-plus, on the other hand, uses a sodium bicarbonate buffer system, wherein the pH varies with $CO_2$ partial pressure.

5) The present composition does not require a mixing step prior to application, and therefore, it is easy to use.

While the invention has been described with respect to certain specific embodiments, it will be appreciated that many modifications and changes may be made by those skilled in the art without departing from the invention.

## Claims

1. An isotonic irrigating solution of pH 7.3 to 7.5 having an osmolarity in the range of from 285 to 300 mOsM, comprising substantially bicarbonate free aqueous phosphate-buffered balanced salt solution, 5 to 10 mM glucose, and 5 to 30 mM β-hydroxybutyrate or a similarly effecitve amount of other ketone bodies and/or precursors thereof so as to form a composition that effectively meets requirements of ocular and other peripheral tissues under surgery for efficient physiological and biochemical functions, but lacking essential amino acids and vitamins that would reduce storage stability.

2. A solution as claimed in Claim 1, wherein the ketone bodies are β-hydroxybutyrate and acetoacetate ions.

3. A solution as claimed in Claim 2, wherein the β-hydroxybutyrate ions are selected from the group consisting of D-isomer of β-hydroxybutyrate, a D- and L-racemic mixture of β-hydroxybutyrate and D- and L-isomer mixtures of β-hydroxybutyrate.

4. A solution as claimed in any preceding claim, wherein the ketone body precursors are short-chain fatty acids selected from the group consisting of acetic acid and butyric acid, or ketogenic amino acids selected from the group consisting of leucine, lysine, phenylalanine, tyrosine and tryptophan.

5. A solution as claimed in Claim 4, wherein composition contains ketone body precursors at a concentration in the range of 0.1-25 mM.

6. A solution as claimed in Claim 4, comprising one or more ketogenic amino acids each having a concentration in the range of 0.1-5.0 mM.

7. A solution as claimed in Claim 4, wherein the ketogenic amino acids have a combined total concentration of 7.5-12.5 mM.

8. A solution as claimed in Claim 4, wherein the short-chain fatty acids are acetic acid at a concentration in the range of 15-25 mM, or butyric acid at a concentration in the range of 5-15 mM.

9. A solution as claimed in Claim 1, wherein a portion of NaCl, in an amount of 15 to 35 mM, is replaced with an equivalent amount of Na salts of one or more sugar acids selected from the group consisting of D-glucuronic acid, D-galacturonic acid, D-mannuronic acid, D-gluconic acid and D-glucaric acid.

10. A solution as claimed in Claim 1, wherein viscosity of the composition is increased by addition of neutral polysaccharide dextrans with mass ranging from 40 to 500 kilodaltons in an amount of from 2 to 20% to form a composition that effectively meets requirements of ocular tissues for efficient physiological and biochemical functions with concurrent prevention of synechia in ocular surgery.

11. A cornea storage medium of pH 7.1 to 7.5, which is substantially free of bicarbonate and which comprises

phosphate buffered balanced salt solution,
5-10 mM glucose,
5-30 mM ketone bodies and or precursors thereof,
20 to 30 mM hepes buffer, and
preformulated minimum essential amino acids and vitamins, wherein the concentration of NaCl is limited to 70 to 100 mM and the osmolarity is adjusted to lie in the range of from 285 to 300 mOsM by the addition of mannitol, sucrose or dextran.

**12.** A cornea storage medium as claimed in Claim 11, wherein the NaCl concentration is no more than 85 mM.

**13.** A cornea storage medium as claimed in Claim 11, wherein the osmolarity of the solution is adjusted to an isotonicity of 290 mOsM.

**14.** A cornea storage medium as claimed in Claim 11, wherein a portion of NaCl, in an amount of 15 to 35 mM, is replaced with an equivalent amount of Na salts of one or more sugar acids selected from the group consisting of D-glucuronic acid, D-galacturonic acid, D-mannuronic acid, D-gluconic acid and D-glucaric acid.

**15.** A cornea storage medium as claimed in Claim 11, further comprising a synergistically effective mixture of VEGF, uridine, thymidine and serum-derived factor.

**16.** A formulation for the preparation of tissues for organ transplant comprising a solution as claimed in Claim 1 and a synergistically effective mixture of VEGF, uridine, thymidine and serum-derived factor.

**17.** A formulation as claimed in Claim 16, for the preparation of tissues for cornea transplant.

**18.** An irrigation solution comprising a cornea storage medium as claimed in Claim 11 and a synergistically effective mixture of VEGF, uridine, thymidine and serum-derived factor.

**19.** An organ culture medium comprising a cornea storage medium as claimed in Claim 11 and a synergistically effective mixture of VEGF, uridine, thymidine and serum-derived factor.

**Patentansprüche**

**1.** Isotonische Berieselungs-(Spül-)Losung mit einem pH-Wert von 7,3 bis 7,5 und einer Osmolarität im Bereich von 285 bis 300 mOsmol, die im wesentlichen hydrogencarbonatfreie wäßrige phosphatgepufferte ausbilanzierte Salzlösung, 5 bis 10 mMol Glucose sowie 5 bis 30 mMol β-Hydroxyburyrat oder eine ähnlich wirksame Menge an anderen Ketonkörpern und/oder Vorlauferverbindungen davon umfaßt, so daß eine Zusammensetzung gebildet wird, die in wirksamer Weise die Erfordernisse von Augen- und anderen peripheren Geweben während der Chirurgie im Hinblick auf effiziente physiologische und biochemische Funktionen erfüllt, aber keine essentiellen Aminosäuren und Vitamine aufweist, die die Lagerstabilität vermindern würden.

**2.** Lösung nach Anspruch 1, worin die Ketonkörper in Form von β-Hydroxybutyrat- und Acetessigsäure-Ionen vorliegen.

**3.** Lösung nach Anspruch 2, worin die β-Hydroxybutyrationen aus der aus dem D-Isomeren von β-Hydroxybutyrat, einem D- und L-Razematgemisch von β-Hydroxybutyrat und den D- und L-Isomergemischen von β-Hydroxybutyrat bestehenden Gruppe ausgewählt sind.

**4.** Lösung nach einem der vorstehenden Ansprüche, worin die Verläuferverbindungen von Ketonkörpern kurzkettige Fettsäuren darstellen, die aus der aus Essigsäure und Buttersäure oder ketogenen Aminosäuren bestehenden Gruppe ausgewählt sind, die ihrerseits aus der aus Leucin, Lysin, Phenylalanin, Tyrosin und Tryptophan bestehenden Gruppe ausgewählt sind.

**5.** Lösung nach Anspruch 4, worin die Zusammensetzung Vorläuferverbindungen von Ketonkörpern in einer Konzentration im Bereich von 0,1 bis 25 mMol enthält.

**6.** Lösung nach Anspruch 4, die eine oder mehrere ketogene Aminosäuren in einer Konzentration von jeweils im Bereich von 0,1 bis 5,0 mMol aufweist.

**7.** Lösung nach Anspruch 4, worin die ketogenen Aminosäuren in der Kombination eine Gesamtkonzentration von 7,5 bis 12,5 haben.

**8.** Lösung nach Anspruch 4, worin die kurzkettigen Fettsäuren in Form von Essigsäure in einer Konzentration im Bereich von 15 bis 25 mMol oder Buttersäure in einer Konzentration im Bereich von 5 bis 15 mMol vorliegen.

**9.** Losung nach Anspruch 1, worin der Anteil an NaCl in einer Menge von 15 bis 35 mMol durch eine äquivalente Menge an Natriumsalzen einer oder mehrerer Zuckersäuren versetzt ist, die aus der aus D-Glucuronsäure, D-Galacturonsäure, D-Mannuronsäure, D-Gluconsäure und D-Glucarsäure bestehenden Gruppe ausgewählt sind.

10. Lösung nach Anspruch 1, worin die Viskosität der Zusammensetzung durch die Zugabe neutraler Polysaccariddextrane mit einer Masse im Bereich von 40 bis 500 Kilodalton in einer Menge von 2 bis 20% zur Bildung einer Zubereitung erhöht wird, die in effektiver Weise die Erfordernisse von Augengeweben im Hinblick auf effiziente physiologische und biochemische Funktionen bei gleichzeitiger Verhütung einer Synechie bei der ophthalmologischen Chirurgie erfüllt.

11. Medium (Flüssigkeit) zur Lagerung von Hornhaut mit einem pH-Wert von 7,1 bis 7,5, das im wesentlichen von Hydrogencarbonat frei ist und das

    eine phosphatgepufferte ausbilanzierte Salzlösung,
    5 bis 10 mMol Glucose,
    5 bis 30 mMol Ketonkörper oder Vorläuferverbindungen davon,
    20 bis 30 mMol Hepespuffer sowie ein
    vorformuliertes Minimum an essentiellen Aminosäuren und Vitaminen aufweist, worin die Konzentration an NaCl auf 70 bis 100 mMol begrenzt und die Osmolarität so eingestellt ist, daß sie durch die Zugabe von Mannit, Saccharose oder Dextran im Bereich zwischen 285 und 300 mOsmol liegt.

12. Medium (Flüssigkeit) zur Lagerung von Hornhaut nach Anspruch 11, worin die NaCl-Konzentration nicht höher ist 85 mMol ist.

13. Medium (Flüssigkeit) zur Lagerung von Hornhaut nach Anspruch 11, worin die Osmolarität der Lösung auf eine Isotonizität von 290 mOsmol eingestellt ist.

14. Medium (Flüssigkeit) zur Lagerung von Hornhaut nach Anspruch 11, worin ein Anteil an NaCl in einer Menge von 15 bis 35 mMol durch eine äquivalente Menge an Natriumsalzen einer oder mehrerer Zuckersäuren ersetzt ist, die aus der aus D-Glucuronsäure, D-Galacturonsäure, D-Mannuronsäure, D-Gluconsäure und D-Glucarsäure bestehenden Gruppe ausgewählt sind.

15. Medium (Flüssigkeit) zur Lagerung von Hornhaut nach Anspruch 11, das zusätzlich noch ein synergistisch wirksames Gemisch aus VEGF (vascular endothelial cell growth factor), Uridin, Thymidin und einem von Serum abgeleiteten Faktor enthält.

16. Formulierung zur Präparierung von Geweben für ein Organtransplantat, das eine Lösung nach Anspruch 1 sowie ein synergistisch wirksames Gemisch aus VEGF, Uridin, Thymidin und einem von Serum abgeleiteten Faktor enthält.

17. Formulierung nach Anspruch 16 zur Präparierung von Geweben für ein Cornea-Transplantat.

18. Berieselungs-(Spül-)Lösung mit einem Gehalt an einem Medium (Flüssigkeit) zur Lagerung von Hornhaut nach Anspruch 11 und einem synergistisch wirksamen Gemisch aus VEGF, Uridin, Thymidin und einem von Serum abgeleiteten Faktor:

19. Organkulturmedium mit einem Gehalt an einem Medium (Flüssigkeit) zur Lagerung von Hornhaut nach Anspruch 11 und einem synergistisch wirksamen Gemisch aus VEGF, Uridin, Thymidin und einem von Serum abgeleiteten Faktor.

**Revendications**

1. Solution d'irrigation isotonique à pH 7,3 à 7,5 ayant une osmolarité dans la gamme de 285 à 300 mOsM, comprenant une solution saline aqueuse équilibrée à tampon phosphate substantiellement exempte de bicarbonate, du glucose 5 à 10 mM et du $\beta$-hydroxybutyrate 5 à 30 mM ou une quantité efficace semblable d'autres matières cétoniques et/ou de précurseurs de celles-ci de façon à former une composition qui répond efficacement aux besoins des tissus oculaires et autres tissus périphériques que l'on opère, pour l'efficacité de leurs fonctions physiologiques et biochimiques, mais n'ayant pas d'acides aminés essentiels ni de vitamines qui réduiraient la stabilité au stockage.

**2.** Solution selon la revendication 1, dans laquelle les matières cétoniques sont des ions β-hydroxybutyrate et acétoacétate.

**3.** Solution selon la revendication 2, dans laquelle les ions β-hydroxybutyrate sont choisis dans le groupe constitué par l'isomère D du β-hydroxybutyrate, un mélange racémique des isomères D et L du β-hydroxybutyrate, et des mélanges des isomères D et L du β-hydroxybutyrate.

**4.** Solution selon l'une quelconque des revendications précédentes, dans laquelle les précurseurs de matières cétoniques sont des acides gras à chaîne courte choisis dans le groupe constitué par l'acide acétique et l'acide butyrique, ou des acides aminés cétogènes choisis dans le groupe constitué par la leucine, la lysine, la phénylalanine, la tyrosine et le tryptophane.

**5.** Solution selon la revendication 4, dans laquelle la composition contient les précurseurs de matières cétoniques à une concentration dans la gamme de 0,1 à 25 mM.

**6.** Solution selon la revendication 4, comprenant un ou plusieurs acides aminés cétogènes ayant chacun une concentration dans la gamme de 0,1 à 5,0 mM.

**7.** Solution selon la revendication 4, dans laquelle les acides aminés cétogènes ont une concentration combinée totale de 7,5 à 12,5 mM.

**8.** Solution selon la revendication 4, dans laquelle les acides gras à chaîne courte sont l'acide acétique à une concentration dans la gamme de 15 à 25 mM, ou l'acide butyrique à une concentration dans la gamme de 5 à 15 mM.

**9.** Solution selon la revendication 1, dans laquelle une partie de NaCl, en une concentration de 15 à 35 mM, est remplacée par une quantité équivalente de sels de Na d'un ou plusieurs acides de sucre choisis dans le groupe constitué par l'acide D-glucuronique, l'acide D-galacturonique, l'acide D-mannuronique, l'acide D-gluconique et l'acide D-glucarique.

**10.** Solution selon la revendication 1, dans laquelle la viscosité de la composition est augmentée par l'addition de polysaccharides dextranes neutres ayant une masse allant de 40 à 500 kilodaltons en une proportion de 2 à 20 % pour former une composition qui répond efficacement aux besoins des tissus oculaires pour l'efficacité de leurs fonctions physiologiques et biochimiques, avec une prévention concomitante de synéchie lors d'une opération chirurgicale oculaire.

**11.** Milieu de conservation pour cornée à pH 7,1 à 7,5, qui est substantiellement exempt de bicarbonate et qui comprend

une solution saline équilibrée à tampon phosphate,
du glucose 5 à 10 mM,
des matières cétoniques 5 à 30 mM ou des précurseurs de celles-ci,
du tampon hepes 20 à 30 mM, et
des acides aminés essentiels et vitamines préformulés minimaux, dans lequel la concentration en NaCl est limitée à 70 à 100 mM et l'osmolarité est reglée pour se trouver dans la gamme de 285 à 300 mOsM par l'addition de mannitol, de saccharose ou de dextrane.

**12.** Milieu de conservation pour cornée selon la revendication 11, dans lequel la concentration en NaCl n'est pas supérieure à 85 mM.

**13.** Milieu de conservation pour cornée selon la revendication 11, dans lequel l'osmolarité de la solution est réglée à une isotonicité de 290 mOsM.

**14.** Milieu de conservation pour cornée selon la revendication 11, dans lequel une partie de NaCl, en une concentration de 15 à 35 mM, est remplacée par une quantité équivalente de sels de Na d'un ou plusieurs acides de sucre choisis dans le groupe constitué par l'acide D-glucuronique, l'acide D-galacturonique, l'acide D-mannuronique, l'acide D-gluconique et l'acide D-glucarique.

**15.** Milieu de conservation pour cornée selon la revendication 11, comprenant en outre un mélange de VEGF (facteur de croissance endothéliale vasculaire), d'uridine, de thymidine et de facteur dérivé du sérum efficaces en synergie.

16. Formulation pour la préparation de tissus pour la transplantation d'organe, comprenant une solution selon la revendication 1 et un mélange de VEGF, d'uridine, de thymidine et de facteur dérivé du sérum efficaces en synergie.

17. Formulation selon la revendication 16, pour la préparation de tissus pour transplantation de cornée.

18. Solution d'irrigation comprenant un milieu de conservation pour cornée tel que revendiqué dans la revendication 11 et un mélange de VEGF, d'uridine, de thymidine et de facteur dérivé du sérum efficaces en synergie.

19. Milieu de culture pour organe, comprenant un milieu de conservation pour cornée tel que revendiqué dans la revendication 11, et un mélange de VEGF, d'uridine, de thymidine et de facteur dérivé du sérum efficaces en synergie.

FIG. I

# FIG. 2

FIG. 3

FIG. 4

FIGURE 6

DETURGESCENCE OF ISOLATED CORNEAS IN BSS-PLUS IN VITRO

FIGURE 5

DETURGESCENCE OF ISOLATED CORNEAS IN THE NOVEL COMPOSITION IN VITRO

FIGURE 8

IN VIVO DETURGESCENCE
OF DONOR CORNEAS
STORED IN OPTISOL

Corneal Thickness (x10², µm)

Post-Operation Duration (days)

7-days storage

11-days storage

FIGURE 7

IN VIVO DETURGESCENCE
OF DONOR CORNEAS
STORED IN MODIFIED
TC199 CONTAINING THE
NOVEL COMPOSITION

Corneal Thickness (x10², µm)

Post-Operation Duration (days)

7-days storage

11-days storage